# EUROPEAN PATENT SPECIFICATION

(11) **EP 3 595 726 B1**
(45) Date of publication and mention of the grant of the patent: **04.10.2023**
(21) Application number: 18724608.7
(22) Date of filing: 19.03.2018
(51) Int. Cl.: A61K 33/38, A61K 45/06, A61P 31/22, A61K 9/00, A61K 47/59

(54) **TREATMENT OF HERPES SIMPLEX SYMPTOMS ON SKIN AND MUCOUS MEMBRANE OF MAMMALS**
BEHANDLUNG VON HERPES-SIMPLEX-SYMPTOMEN AUF DER HAUT UND SCHLEIMHAUTMEMBRAN VON SÄUGETIEREN
TRAITEMENT DES SYMPTÔMES DE L'HERPÈS SIMPLEX SUR LA PEAU ET LES MEMBRANES MUQUEUSES DE MAMMIFÈRES

(30) Priority: 17.03.2017 FI 20175246
(43) Date of publication of application: 22.01.2020
(73) Proprietor: Nieminen, Jyri, Escaldes-Engordany AD700 (AD)
(72) Inventor: NIEMINEN, Jyri, Escaldes-Engordany (AD)
(74) Representative: Laine IP Oy
(86) International application number: PCT/FI2018/050202
(87) International publication number: WO 2018/167379

(56) References cited:
- EP-A1- 0 326 145
- WO-A1-02/30204
- WO-A1-2013/026961
- V. R. COLEMAN ET AL: "Inactivation of Herpesvirus hominis Types 1 and 2 by Silver Nitrate In Vitro and In Vivo", ANTIMICROBIAL AGENTS AND CHEMOTHERAPY, vol. 4, no. 3, 1 September 1973 (1973-09-01), pages 259-262, XP055492964, US ISSN: 0066-4804, DOI: 10.1128/AAC.4.3.259
- STEFANIA GALDIERO ET AL: "Silver Nanoparticles as Potential Antiviral Agents", MOLECULES, vol. 16, no. 10, 24 October 2011 (2011-10-24), pages 8894-8918, XP055492993, DOI: 10.3390/molecules16108894
- DONG XU ET AL: "Polyethyleneimine Capped Silver Nanoclusters as Efficient Antibacterial Agents", INTERNATIONAL JOURNAL OF ENVIRONMENTAL RESEARCH AND PUBLIC HEALTH, vol. 13, no. 3, 18 March 2016 (2016-03-18) , page 334, XP055493271, DOI: 10.3390/ijerph13030334

## Description

### Technical Field

The present invention relates to a composition as defined in the appended claims for use in the medical treatment of herpes symptoms, in particular herpes simplex symptoms, such as sores, wherein the composition is applied topically on skin and mucous membrane of mammals. The invention also relates to a method of manufacturing such compositions.

### Background

There are two types of herpes simplex virus. HSV-1 causes cold sores on the lips and on mucous membrane of the mouth. HSV-1 can also cause sores around the genitals. HSV-2 causes sores in the genital area. Globally approximately two-thirds of people under the age of 50 have herpes, according to WHO. The highest rates are found in Africa, South-East Asia and the Western Pacific, but nearly 60 percent of U.S. men and women between the ages of 14 and 49 are carrying the HSV-1 virus. About 16 to 25 percent of U.S. men and women between 14 and 49 are infected with HSV-2. Herpes is a lifelong infection and makes infected 2-3 times more likely to acquire HIV. The herpes infection is also often called fever blisters, cold sores, herpes catarrhalis, herpes facialis, herpes febrilis, herpes genitalis, herpes labialis, herpes mentalis, herpes preputialis, and herpes progenitalis. According to WHO, herpes virus is a major global problem and current medicines are not working properly. During the last two decades, state of the art antiviral medication has been acyclovir, valacyclovir and similar, which affect slowly, and in topical treatments 5 to 10 days are needed for healing. Slow healing of outbreaks is a big problem due to pain, social shame, possibility to infect other people and possible secondary bacterial infections. Due to low bioavailability of common antiviral acyclovir, multiple and repetitive applications per day are needed, such as five pills per day for five days or applying of ointment 5-6 times a day. Acyclovir, valacyclovir and related antivirals are not efficient enough to provide a quick relief of the pain, burning, tingling and itching, and it takes several days to have an effect.

In addition to numerous acyclovir and valacyclovir related patents, various other treatments of herpes simplex have been proposed. US 4,147,803 discloses the topical application of lauric diethanolamide to the area affected. Application of a mixture of boric acid, tannic acid, and salicylic acid is taught in US 4,285,934. The use of lignosulfonate as a topical treating agent is disclosed in US 4,185,097, and the application of kelp to the affected area is proposed in US 4,117,120.

There is a significant, unmet need for new approaches for suppressing and treating herpes infections.

### Summary of the Invention

It is an object of the present invention to provide a new composition for use in the treatment of herpes simplex in mammals.

The present invention is based on the concept of preparing a pharmaceutical topical composition for use in the treatment of infections caused by herpes virus, comprising ionic silver present in the form of a polymeric complex.

The composition can be applied topically on the skin of a subject who suffers from an infection caused by a herpes virus, in particular a herpes simplex virus.

Thus, in a first aspect, there is provided a composition for use in the treatment of a herpes virus infection, in particular a herpes simplex virus infection, wherein the composition is used as an antiviral medicament and is applied topically to areas on the mammal skin or mucous membrane affected with a herpes virus, in particular herpes simplex virus, and wherein said composition comprises a ionomer composition of silver ions complexed with polyethyleneimine.

In a second non-claimed aspect, there is provided a pharmaceutical topical composition for use in the treatment of a herpes virus infection, wherein the composition comprises a virucidal molecule like acyclovir or valacyclovir or similar and polyethyleneimine complexing ionic silver. Different action mechanisms are having synergetic effects.

More specifically, the compositions for use according to the present invention are characterized by what is stated in claim 1.

The method according to the invention is characterized by what is stated in claim 12.

Considerable advantages are obtained by the invention. Thus, the present invention helps to prevent herpes cold sores or even prevents outbreaks thereof. It shortens healing time of cold sores/outbreaks, provides a quick relief for the pain, burning and itching of cold sores/outbreaks and limits the risk of transmission of the infection to others or exposure to a secondary infection.

As will be discussed above, disappearance of symptoms including cold sores and lesions will be achieved within about 2 to 3 days, and relief of pain from the affected area, e.g. blisters, will be achieved within 10 to 15 minutes of the first application depending upon the size of the lesions or cold sores.

These and other objects and advantages of the invention will be discussed in connection with embodiments of the present invention, discussed in the following.

### Embodiments

In the present context, the abbreviation "HSV" is used for denoting herpes simplex and "HSV-1" for herpes simplex virus type I and "HSV-2" for herpes simplex virus type II. Further, "VZZ" stands for varicella zoster virus, "EBV" for epstein-barr virus and "CMV" and "hCMV" for cytomegalovirus and for human cytomegalovirus, respectively. "HPV" stands for human papilloma virus.

According to the present invention, symptoms caused by virus, such as HSV-1 and HSV-2 symptoms, for example cold sores, can be treated on skin and mucous membrane with silver polymer formulation of this innovation to solve the most of the drawbacks of today's state of the art medications.

According to Coleman et al (1973) silver nitrate instillation into the eyes of a newborn infected with HSV might prevent eye infection with HSV type 1. Said publication does not disclose a composition for use in the treatment of herpes virus infection, wherein said composition comprises a ionomer composition of silver ions complexed with polyethyleneimine.

Antimicrobial ionomer compositions comprising amine functional cationic polymer and ionic silver have been disclosed in WO 2013/026961 A1. Said publication is silent about the potential use of the compositions for antiviral therapy.

Surprisingly, silver polymer formulations as disclosed herein heal cold sores and outbreaks in 2 to 3 days and when tickling areas are treated before formation of cold sores, surprisingly the cold sores are not breaking out. Formulation gives fast and remarkable relief for itching and pain of cold sores. Silver polymer formulation is shown to be highly antiviral in *in vitro* tests, and it is killing effectively viral viruses in cold sores *in vivo.* This surprising effectivity remarkably limits the suffering of subjects infected with herpes and the possibility to infect others.

This application relates to compositions for use in the treatment of herpes infection on skin or mucous membrane, occurring in humans and animals or mammals, and provides effective topical formulations, dosage forms and dosing regimens improvably effective for arresting and treating such skin outbreaks, especially those caused by hCMV, HSV-1, HSV-2. Infections to be treated include genital herpes, herpes zoster, chicken pox and warts (HPV). The skin, eye, ear, nose, mouth and mucosal applications can be targeted with these formulations.

The pharmaceutical topical composition for use according to the invention comprises a ionomer composition of silver ions complexed with polyethyleneimine.

In a preferred embodiment of the invention, said polyethyleneimine comprises a branched polyethyleneimine, a linear polyethyleneimine or a mixture of corresponding polyethyleneimines of different qualities with different properties with regard to e.g. molecular weights and primary:secondary:tertiary -amine ratios. Also copolymers of polyethyleneimines are useful.

The polyethyleneimine preferably comprises or consists of a potentially branched poly(ethyleneimine) having a molecular weight (Mw) between 200 and 3,000,000, in particular about 750 to 2,000,000.

In a preferred embodiment of the invention, the silver ion source is silver halide, silver oxide, silver nitrate or metallic silver. In some embodiments, the silver ion source may be silver acetate, silver stearate, silver saccharinate, silver imidazole, silver citrate, silver methacrylate and silver sulfadiazine.

In a further preferred embodiment of the invention, the pharmaceutical topical composition for use according to the invention further comprises a solvent, a solvent mixture, or a solvent matrix. The solvent is preferably a hydroxyl group containing solvent, such as water, ethanol, 1,3-butanediol, methyl, propyl or butyl alcohol or combinations thereof.

In a second non-claimed aspect, there is provided a pharmaceutical topical composition for use according to the invention comprising a virucidal molecule like acyclovir or valacyclovir or similar and polyethyleneimine complexing ionic silver. Different action mechanisms are having synergetic effects.

It has been found that herpes infections, in particular herpes simplex infections, can be effectively treated by topical application to the affected area of an effective amount of a composition comprising polyethyleneimine complexing ionic silver. It has been found that application of 0.01 to 1 ml of polyethyleneimine complex containing about 0.5 to 1.0 mg of ionic silver is an effective amount. The polyethyleneimine silver complex is preferably diluted in a solvent or solvent mixture comprising hydroxy containing solvents like butyl glycol, ethanol etc. In addition to the active ingredients mentioned above the composition may contain a further antiviral substance, for example acyclovir, valacyclovir, penciclovir, famciclovir or similar first-line antiviral agent or combinations thereof.

The composition of this invention contains 10 to 100,000 ppm of ionic silver, and about 100 to 999,000 ppm of polyamine polymer, more preferably 100 to 10,000 ppm of ionic silver and 500 to 100,000 ppm of polyamine polymer. In a further embodiment the composition contains less than 7500 ppm of ionic silver.

The formulation may further contain a stabiliser component like saccharine or aminomethyl propanol.

The topical compositions for use according to the invention can be for example in the form of cream, ointment, lotion, liquid, fluid, spray or foam, having a silver concentration of for example 5-1000 ppm. The pH of the formulation is preferably from 7.5 to 11.5.

The treatment of the herpes simplex infection in accordance with this invention comprises topical application of the composition disclosed herein to the affected area on the person suffering from the infection.

Generally, disappearance of symptoms including cold sores and lesions will be achieved within about 2 to 3 days. The relief of pain from the affected area will be achieved within 10 to 15 minutes after the first application depending upon the size of the lesions or cold sores when treatment is started.

In one embodiment, an effective amount of the composition 0.05 to 0.10 ml, containing about 0.50 to 1.00 mg of ionic silver per application, is applied to the affected area 1 to 4 times a day until healing is achieved.

Typically the area treated is about 0.01 to 200 cm², for example 0.05 to 100 cm², in particular about 0.1 to 50 cm², such as 0.15 to 10 cm² or 0.2 to 5 cm², or 0.25 to 2 cm² and the applied amount of ionic silver is about 0.01 to 10 mg/cm², for example about 0.1 to 5 mg/cm², typically per application.

The composition for use in accordance with the present invention is effective in the treatment of all areas on the mammal body affected by a herpes virus, for example herpes simplex virus, such as cold sores, lesions, warts and blisters. Mucous membranes in mouth and genital area are more sensitive compared skin and lips, and lower polyethyleneimine silver ion complex concentrations can be effectively used to minimize irritation of mucous membrane.

The following non-limiting examples represent interesting embodiments.

### Example 1

An antiviral composition was produced in a solvent matrix. 2.0 grams of a branched polyethyleneimine (Lupasol WF, BASF, MW 25 000) was solubilized in 8 grams of 1,3-butanediol and cooled down. The solution was reacted together with 0.295 grams of silver chloride by mixing said suspension at room temperature until a clear solution was formed. The obtained ionomer composition had a dry content (mass of the solvent excluded) of 22.3 % (w/w) and theoretical silver content of 9.7 % (w/w) of dry mass.

### Example 2

An antiviral ionomer composition was produced in a solvent matrix. Approximately 2.0 g of a branched polyethyleneimine (Lupasol G20 waterfree, BASF, molecular weight 1300) was mixed with 6 g of ethyl alcohol and cooled down. The solution was reacted with 0.724 g of silver saccharinate by mixing said suspension at room temperature until a clear solution was formed. The process was continued by adding of 2.49 ml of 1 M hydrochloric acid into said solution under continuous mixing. A clear solution of an optically clear ionomer composition was formed, having a dry content (w/w) of 25.0 % and theoretical silver content of 9.5 % (w/w) of dry mass.

### Example 3

An antiviral ionomer composition was produced in a solvent matrix. 1.0 grams of a branched polyethyleneimine (Lupasol G20 waterfree, BASF, molecular weight 1300) was solubilized to 3 grams of ethyl alcohol and cooled down. The solution was reacted together
with 0.156 grams of silver saccharinate by mixing said suspension at room temperature until a clear solution was formed. The process was continued by diluting the intermediate composition to a total volume of 50 ml with EtOH. Finally, the product was chloridized by adding 0.570 ml of 1 M HCl under mixing conditions.

### Example 4

An antiviral ionomer composition was produced in a solvent matrix. 3.0 grams of a branched polyethyleneimine (Lupasol PS, BASF, molecular weight 750,000, concentration in water 33%) was mixed with 1 grams of ethyl alcohol and cooled down. The solution was reacted together with 0.156 grams of silver saccharinate by mixing said suspension at room temperature until a clear solution was formed. The intermediate composition was diluted to a total volume of 50 ml with EtOH. Finally, the composition was chloridized by adding 0.570 ml of 1 M HCl under mixing conditions.

### Example 5

An antiviral ionomer composition was produced in a solvent matrix. 2.0 grams of a branched polyethyleneimine (Lupasol WF, BASF, MW 25,000) was solubilized in 8 grams of ethyl alcohol and cooled down. The solution was reacted together with 0.295 grams of silver chloride by mixing said suspension at room temperature until a clear solution was formed. The process was continued by adding 0.378 grams of saccharin and mixing until a clear solution was formed.

### Example 6

An antiviral ionomer composition was produced in a solvent matrix. 2.0 grams of a branched polyethyleneimine (Lupasol P, BASF, MW 750,000, concentration in water 50 %) was mixed with 5.460 grams of ethyl alcohol and cooled down. The solution was reacted together with 0.345 grams of silver chloride by mixing said suspension at room temperature until a clear solution was formed. The process was continued by adding 0.5 grams of saccharin. An ionomer composition with 14.1% w/w theoretical silver content (of dry weight) was produced.

### Application Example 7

Antiviral performance of the composition produced as described in Example 1 was screened *in vitro* according to the European Standard EN 14476:2013 for a herpes simplex virus type 1 (HSV-1). Said composition was added to a suspension of herpes viruses in an interfering substance (clean conditions) in concentrations of 80 % and 97 %. The mixture was maintained at 20 °C for 60 seconds and 5 min. At the end of the exposure time, aliquots were taken, and the virucidal action was neutralised by transferring the aliquots into cold diluents. Serial dilutions were performed and assayed for residual infectivity. All viruses were found dead.

### Application Example 8

Antiviral performance of the composition of the present invention was evaluated *in* vivo.The test group A consisted of four individuals suffering from herpes simplex labialis. All members of the group had a long history with herpes infection and all members had been testing all available herpes medications in the market. As infections were untreated, the individuals were normally healing in between 7 to 10 days. The time from initiation of the symptoms to when the patients experienced relief of pain from the affected area was between 5 to 7 days. As infections were treated with common topical acyclovir medication 5 to 6 times per day, healing resulted in between 5 to 7 days. The time from initiation of the symptoms to time when the patients experienced relief of pain from the affected area was 3 to 5 days. As infections were treated with common topical valacyclovir medication, recovery was similar compared acyclovir.

The herpes infections of test group were treated topically with a composition containing the polyethyleneimine silver complex with the following results. The composition was applied topically to the affected areas to cover the cold sores with a coating of the composition two times a day. The time from initiation of the cold sores and treatment with the composition to complete healing averaged between about 2 to 3 days. The time from the initiation of the treatment to when the patients experienced relief of pain from the affected area was about 10 to 15 minutes after the first application. As the composition was applied topically to the affected areas as the first sensations of herpes outbreak appears, the cold sores didn't appear at all. The composition for use according to the present invention surprisingly prevents cold sores to develop when formulation is applied as the first sensations of outbreak appear.

### Application Example 9

In another *in vivo* test, the test group B consisted of three individuals suffering from oral herpes simplex. As infections were untreated, the individuals resulted in complete healing normally in 8 to 11 days. The time from initiation of the symptoms to when the patients experienced relief of pain from the affected area was normally 6 to 7 days.

As the infections of test group B were treated topically with a composition containing the polyamine polymer silver complex with the following results. The composition was applied topically to the cold sores to cover the affected areas with a layer of the composition two times a day. The time from initiation of the cold sores and treatment with the composition to healing was about two days. The time from the initiation of the treatment to when the patients experienced relief of pain from the affected area was about 10 to 15 minutes.

The composition for use according to the invention is reducing the average time for healing about 70 % compared to untreated herpes infections. More significantly, treatment with the composition results in relief of pain from the affected areas in about 500 to 1000 times faster than in untreated infections. More significantly, the composition for use according to the present invention surprisingly prevents cold sores or lesions to appear as formulation is applied at the first signs of tingling, burning or itching. More significantly, any member of test group A or B were not willing to stop using of the composition after the test period.

*In* vivo testing has shown that the compositions for use according to the present invention are effective within the broad range of concentrations. The preferred range of concentration for silver is 100 ppm to 100 000 ppm, more preferably 300 to 7 500 ppm.

The compositions for use according to the present invention can be used together with common antiviral molecules like acyclovir or valacyclovir or similar. Polyethyleneimine silver ion complex is boosting performance of antiviral molecules remarkably. PEI is suitable carrier or delivery mechanism for acyclovir. PEI is distributing acyclovir molecules perfectly due to attraction forces between PEI molecule and capsid of herpes virus.

### Industrial Applicability

The present compositions can be used for treatment of herpes simplex symptoms on skin and mucous membranes of mammals, such as humans, by topical application of a medical composition. The treatment can be combined with other forms of therapy and with the use of first-line and second line antiviral agents for treatment of, in particular HSV-1 and HSV-2 infections and with symptoms caused by such infections. Other virus infections which can be treated with the present invention include infections caused by CMV, hCMV, VZZ, EBV and HPV. As discussed above, the compositions can be formulated for application on skin, eye, ear, nose, mouth and mucous membranes.

### Citation List

Coleman et al, Inactivation of Herpesvirus hominis Types 1 and 2 by Silver Nitrate In Vitro and In Vivo. Antimicrobial Agents and Chemotherapy, Vol. 4, No. 3, 1973, 259-262
US 4,147,803
US 4,285,934
US 4,185,097
US 4,117,120

## Claims

1. A composition for use in the treatment of a herpes virus infection, in particular a herpes simplex virus infection, wherein the composition is used as an antiviral medicament and is applied topically to areas on the mammal skin or mucous membrane affected with a herpes virus, in particular herpes simplex virus, and wherein said composition comprises a ionomer composition of silver ions complexed with polyethyleneimine.

2. The composition for use according to claim 1, wherein the silver ion source is selected from the group of silver halides, silver oxides, silver nitrates and metallic silvers and combinations thereof.

3. The composition for use according to claim 1 or 2, wherein the composition further comprises a solvent or a solvent mixture.

4. The composition for use according to any of the preceding claims, wherein the solvent is compound containing hydroxy groups, such as water or an alcohol, such as ethanol, 1,3-butanediol, or a mixture thereof.

5. The composition for use according to any of the preceding claims, wherein the silver concentration is 10 - 100,000 ppm, in particular 100 - 10,000 ppm, preferably less than 7,500 ppm.

6. The composition for use according to any of the preceding claims, wherein the formulation further contains a stabiliser component, such as saccharine or aminomethyl propanol, or a mixture thereof.

7. The composition for use as claimed in any of the preceding claims, wherein polymer concentration in the formulation is between 1 000 to 100 000 ppm.

8. The composition for use as claimed in any of the preceding claims, wherein the formulation further contains an additional antiviral component, such as acyclovir valacyclovir, penciclovir, famciclovir or combinations thereof, in particular for synergetic action.

9. The composition for use as claimed in any of the preceding claims, wherein pH of the formulation is 7.5 to 11.5.

10. The composition for use as claimed in any of the preceding claims, wherein pH of the formulation is adjusted with hydrochloric acid.

11. The composition for use as claimed in any of the preceding claims, comprising an aqueous or alcoholic ionomer composition of silver and polyethyleneimine, having a pH of 7.5 to 11.5 and containing 1000 to 100.000 ppm of silver.

12. A method of producing an antiviral pharmaceutical composition comprising the steps of mixing silver ions complexed with polyethyleneimine with an antiviral agent.

13. The method according to claim 12, comprising mixing a composition containing silver ions complexed with polyethyleneimine, with an antiviral agent, such as acyclovir, valacyclovir, penciclovir, famciclovir or combinations thereof.

14. The composition for use according to any one of claims 1 to 10, wherein the herpes simplex virus is hCMV, HSV-1 or HSV-2.

## Patentansprüche

1. Zusammensetzung zur Verwendung in der Behandlung einer Herpesvirusinfektion, insbesondere einer Herpes-simplex-Virusinfektion, wobei die Zusammensetzung als ein antivirales Medikament verwendet wird und topisch auf Flächen auf der Säugetierhaut oder Schleimhautmembran aufgetragen wird, die von einem Herpesvirus, insbesondere Herpes-simplex-Virus, befallen ist, und wobei die Zusammensetzung eine ionomere Zusammensetzung aus Silberionen im Komplex mit Polyethylenimin umfasst.

2. Zusammensetzung zur Verwendung nach Anspruch 1, wobei die Silberionenquelle ausgewählt ist aus der Gruppe von Silberhalogeniden, Silberoxiden, Silbernitraten und metallischem Silber und Kombinationen davon.

3. Zusammensetzung zur Verwendung nach Anspruch 1 oder 2, wobei die Zusammensetzung weiter ein Lösemittel oder ein Lösemittelgemisch umfasst.

4. Zusammensetzung zur Verwendung nach einem der vorstehenden Ansprüche, wobei das Lösemittel eine Verbindung ist, die Hydroxygruppen wie Wasser oder einen Alkohol wie Ethanol, 1,3-Butandiol oder ein Gemisch davon enthält.

5. Zusammensetzung zur Verwendung nach einem der vorstehenden Ansprüche, wobei die Silberkonzentration 10- 100.000 ppm, insbesondere 100- 10.000 ppm, bevorzugt weniger als 7.500 ppm ist.

6. Zusammensetzung zur Verwendung nach einem der vorstehenden Ansprüche, wobei die Formulierung weiter eine Stabilisatorkomponente, wie Saccharin oder Aminomethylpropanol oder ein Gemisch davon, enthält.

7. Zusammensetzung zur Verwendung nach einem der vorstehenden Ansprüche, wobei eine Polymerkonzentration in der Formulierung zwischen 1.000 und 100.000 ppm ist.

8. Zusammensetzung zur Verwendung nach einem der vorstehenden Ansprüche, wobei die Formulierung weiter eine zusätzliche antivirale Komponente enthält, wie Acyclovir, Valacyclovir, Penciclovir, Famciclovir oder Kombinationen davon, insbesondere für synergetische Wirkung.

9. Zusammensetzung zur Verwendung nach einem der vorstehenden Ansprüche, wobei ein pH-Wert der Formulierung 7,5 bis 11,5 ist.

10. Zusammensetzung zur Verwendung nach einem der vorstehenden Ansprüche, wobei ein pH-Wert der Formulierung mit Salzsäure eingestellt wird.

11. Zusammensetzung zur Verwendung nach einem der vorstehenden Ansprüche, die eine wässrige oder alkoholische ionomere Zusammensetzung aus Silber und Polyethylenimin mit einem pH-Wert von 7,5 bis 11,5 umfasst und 1.000 bis 100.000 ppm Silber enthält.

12. Verfahren zum Herstellen einer antiviralen pharmazeutischen Zusammensetzung, umfassend die Schritte zum Mischen von Silberionen im Komplex mit Polyethylenimin mit einem antiviralen Mittel.

13. Verfahren nach Anspruch 12, umfassend Mischen einer Zusammensetzung, die Silberionen im Komplex mit Polyethylenimin enthält, mit einem antiviralen Mittel, wie Acyclovir, Valacyclovir, Penciclovir, Famciclovir oder Kombinationen davon.

14. Zusammensetzung zur Verwendung nach einem der Ansprüche 1 bis 10, wobei das Herpes-simplex-Virus hCMV, HSV-1 oder HSV-2 ist.

## Revendications

1. Composition pour l'utilisation dans le traitement d'une infection par virus herpès, en particulier une infection par virus herpès simplex, dans laquelle la composition est utilisée en tant que médicament antiviral et est appliquée topiquement sur des zones sur la peau ou membrane muqueuse mammifère affectée par un virus herpès, en particulier un virus herpès simplex, et dans laquelle ladite composition comprend une composition ionomère d'ions d'argent en complexe avec polyéthylèneimine.

2. Composition pour l'utilisation selon la revendication 1, dans laquelle la source d'ions d'argent est sélectionnée parmi le groupe de : halogénures d'argent, oxydes d'argent, nitrates d'argent et argents métalliques et associations de ceux-ci.

3. Composition pour l'utilisation selon la revendication 1 ou 2, dans laquelle la composition comprend en outre un solvant ou un mélange de solvant.

4. Composition pour l'utilisation selon l'une quelconque des revendications précédentes, dans laquelle le solvant est un composé contenant des groupes hydroxy, tels que de l'eau ou un alcool, tel qu'éthanol, 1,3-butanediol, ou un mélange de ceux-ci.

5. Composition pour l'utilisation selon l'une quelconque des revendications précédentes, dans laquelle la concentration en argent est de 10 à 100 000 ppm, en particulier de 100 à 10 000 ppm, de préférence inférieure à 7 500 ppm.

6. Composition pour l'utilisation selon l'une quelconque des revendications précédentes, dans laquelle la préparation contient en outre un composant stabilisateur, tel que saccharine ou aminométhylpropanol, ou un mélange de ceux-ci.

7. Composition pour l'utilisation selon l'une quelconque des revendications précédentes, dans laquelle la concentration en polymère dans la préparation est entre 1000 et 100 000 ppm.

8. Composition pour l'utilisation selon l'une quelconque des revendications précédentes, dans laquelle la préparation contient en outre un composant antiviral supplémentaire, tel que : acyclovir valacyclovir, penciclovir, famciclovir ou associations de ceux-ci, en particulier pour action synergétique.

9. Composition pour l'utilisation selon l'une quelconque des revendications précédentes, dans laquelle le pH de la préparation est de 7,5 à 11,5.

10. Composition pour l'utilisation selon l'une quelconque des revendications précédentes, dans laquelle le pH de la préparation est ajusté avec de l'acide chlorhydrique.

11. Composition pour l'utilisation selon l'une quelconque des revendications précédentes, comprenant une composition ionomère aqueuse ou alcoolique d'argent et de polyéthylèneimine, présentant un pH de 7,5 à 11,5 et contenant de 1000 à 100 000 ppm d'argent.

12. Procédé de production d'une composition pharmaceutique antivirale, comprenant les étapes du mélangeage d'ions d'argent, en complexe avec polyéthylèneimine, avec un agent antiviral.

13. Procédé selon la revendication 12, comprenant le mélangeage d'une composition contenant des ions d'argent, en complexe avec polyéthylèneimine, avec un agent antiviral, tel que : acyclovir, valacyclovir, penciclovir, famciclovir ou des associations de ceux-ci.

14. Composition pour l'utilisation selon l'une quelconque des revendications 1 à 10, dans laquelle le virus herpès simplex est hCMV, HSV-1 ou HSV-2.
